# FASCICULE DE BREVET EUROPEEN

(11) **EP 1 981 834 B1**
(45) Date de publication et mention de la délivrance du brevet: **02.11.2011**
(21) Numéro de dépôt: 07731583.6
(22) Date de dépôt: 06.02.2007
(51) Int. Cl.: C07C 45/52, C07C 45/35, C07C 51/25, C07C 29/60

(54) **PROCEDE DE PREPARATION D'ACROLEINE**
VERFAHREN ZUR HERSTELLUNG VON ACROLEIN
ACROLEIN PREPARATION METHOD

(30) Priorité: 07.02.2006 FR 0601059
(43) Date de publication de la demande: 22.10.2008
(73) Titulaire: ARKEMA FRANCE, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2007/050757
(87) Numéro de publication internationale: WO 2007/090990

(56) Documents cités:
- WO-A-2004/099113
- DE-C1- 4 238 493
- GB-A- 1 152 215

## Description

### Domaine techniques

La présente invention concerne un procédé de préparation d'acroléine à partir du propylène, comprenant une étape de déshydratation de glycérol en présence d'un gaz contenant du propylène et plus particulièrement en présence du gaz de réaction issu de l'étape d'oxydation du propylène en acroléine.

### Art antérieur et problème technique

Le procédé de production d'acroléine le plus communément utilisé est basé sur la réaction d'oxydation catalytique en phase gazeuse du propylène par l'oxygène de l'air, tel que décrit par exemple dans le document Technique de l'ingénieur, traité Génie des procédés J 6 100 1-4. L'acroléine ainsi obtenue peut, soit, être directement intégrée dans un procédé en deux étapes de fabrication d'acide acrylique à partir du propylène en phase gazeuse, soit, être utilisée comme intermédiaire de synthèse. L'acroléine est en particulier un intermédiaire clé pour la synthèse de la méthionine, acide aminé de synthèse utilisée comme complément de l'alimentation animale qui s'est imposé comme substitut des farines de poisson. L'acroléine trouve aussi de nombreuses autres applications pour préparer des dérivés qui peuvent être synthétisés sur le site même de production de l'acroléine, limitant ainsi le stockage et le transport de ce produit chimique toxique.

Dans un certain nombre de cas, il peut être intéressant de pouvoir augmenter les capacités de production en acroléine des unités existantes.

La production d'acroléine est fortement dépendante de la matière première propylène. Le propylène, obtenu par vapocraquage ou craquage catalytique de coupes pétrolières a l'inconvénient de contribuer à l'augmentation de l'effet de serre, du fait de son origine fossile. Par ailleurs, la ressource en propylène peut devenir limitée.

Il apparaît donc particulièrement intéressant de pouvoir augmenter la productivité en acroléine, tout en réduisant la dépendance à une ressource fossile.

Il est connu depuis longtemps que le glycérol peut conduire à l'obtention d'acroléine. Le glycérol est issu de la méthanolyse des huiles végétales en même temps que les esters méthyliques qui sont, eux, employés notamment comme carburants ou combustibles dans le gazole et le fioul domestique. C'est un produit naturel qui jouit d'une aura "verte", il est disponible en grande quantité et peut être stocké et transporté sans difficulté. De nombreuses études sont consacrées à la valorisation du glycérol selon son degré de pureté, et la déshydratation du glycérol en acroléine est une des voies envisagées.

La réaction mise en jeu pour obtenir l'acroléine à partir du glycérol est :

CH₂OH-CHOH-CH₂OH ⇔ CH₂=CH-CHO + 2H₂O

En règle générale la réaction d'hydratation est favorisée aux basses températures, et la réaction de déshydratation est favorisée aux températures élevées. Pour obtenir l'acroléine, il faut donc mettre en oeuvre une température suffisante, et/ou un vide partiel pour déplacer la réaction. La réaction peut être effectuée en phase liquide ou en phase gaz. Ce type de réaction est connu pour être catalysée par des acides. Différents procédés de synthèse de l'acroléine à partir du glycérol sont décrits dans l'art antérieur ; on peut citer notamment les documents FR 695931, US 2,558,520, WO 99/05085, US 5,387,720. GB 1,152,215 et WO 2004/099113 décrivent un procédé

de préparation d'acroleine par oxydation de propylène. Il a maintenant été trouvé que la réaction de déshydratation du glycérol en acroléine peut s'effectuer en présence d'un gaz contenant du propylène. Il est ainsi avantageux d'introduire du glycérol dans le procédé d'oxydation catalytique en phase gazeuse du propylène, ce qui permet d'utiliser une matière première renouvelable tout en augmentant la production d'acroléine. Un tel procédé devient particulièrement avantageux pour la synthèse de méthionine qui peut alors être dite "obtenue à partir de biomasse". En effet, la méthionine lors de son utilisation dans l'alimentation animale, est métabolisée rapidement et le gaz carbonique qui se retrouve dans l'atmosphère contribue à l'augmentation de l'effet de serre. Si l'acroléine est obtenue partiellement à partir d'une matière première renouvelable, tel que le glycérol provenant d'huile végétale, les émissions de CO₂ n'entrent plus entièrement dans le bilan du procédé, car elles compensent le gaz carbonique utilisé par la biomasse pour sa croissance ; il y a donc une limitation de l'augmentation de l'effet de serre. Un tel procédé répond alors aux critères associés au nouveau concept de "chimie verte" dans un cadre plus global de développement durable.

### Exposé de l'invention

L'objet de la présente invention est donc un procédé de préparation d'acroléine par oxydation du propylène comprenant une étape de déshydratation de glycérol en présence d'un gaz contenant du propylène.

La réaction de déshydratation du glycérol peut être réalisée en présence du mélange gazeux qui alimente le réacteur d'oxydation du propylène, généralement constitué de propylène, de vapeur d'eau, d'un gaz inerte pouvant être de l'azote ou de l'argon, et d'oxygène moléculaire ou d'un gaz contenant de l'oxygène moléculaire.

Selon un mode de réalisation préféré de l'inve ntion, l'étape de déshydratation du glycérol est réalisée en présence du gaz de réaction issu de l'étape d'oxydation du propylène en acroléine. Ce gaz de réaction est généralement constitué d'un mélange des gaz n'ayant pas réagi (propylène non transformé, propane présent initialement dans le propylène, gaz inerte, vapeur d'eau, oxygène, CO, CO₂), d'acroléine produite et de différents sous-produits tels que acide acrylique, acide acétique et autres composés minoritaires.

Sans que la demanderesse soit tenue à une quelconque explication, elle pense que l'étape de déshydratation du glycérol permet de refroidir les gaz de réaction issus de l'étape d'oxydation du propylène en acroléine.

En effet, dans la réaction d'oxydation du propylène en acroléine, les gaz de réaction sortent de la zone de réaction à une température élevée, la réaction d'oxydation du propylène étant exothermique. Il est nécessaire de refroidir ces gaz de réaction pour récupérer l'acroléine. Dans un procédé de préparation d'acide acrylique à partir du propylène en deux étapes, il est nécessaire aussi de refroidir les gaz de réaction issus de la première étape d'oxydation du propylène en acroléine avant d'entrer dans le second étage d'oxydation de l'acroléine en acide acrylique, car la réaction d'oxydation de l'acroléine en acide acrylique s'effectue à une température plus basse que la réaction d'oxydation du propylène en acroléine. De plus, l'acroléine peut s'auto enflammer aux températures élevées entraînant des pertes de rendements.

Ce refroidissement est en général obtenu grâce à un échangeur thermique placé en aval de la zone catalytique. Le même effet peut, en tout ou partie, être obtenu grâce à la mise en oeuvre d'une réaction endothermique, telle que la déshydratation du glycérol. Dans la présente invention, la réaction de déshydratation du glycérol présente l'avantage de conduire au même produit de réaction principal (acroléine) que la réaction d'oxydation du propylène. Il en résulte ainsi une augmentation de la productivité en acroléine tout en récupérant de manière efficace la chaleur de la réaction d'oxydation.

D'autres caractéristiques et avantages de l'invention ressortiront mieux à la lecture de la description qui suit et en référence aux figures annexées dans lesquelles :
- la Figure 1 représente schématiquement un réacteur d'oxydation du propylène en acroléine, conventionnel.
- Les Figures 2, 3, 4 et 5 représentent schématiquement différenties configurations de réacteurs d'oxydation du propylène en acroléine correspondant à des modes de réalisation du procédé selon l'invention.

### Exposé détaillé de l'invention

Dans le procédé de l'invention, l'étape de déshydratation de glycérol est effectuée en phase gaz en présence d'un catalyseur à une température allant de 150°C à 500°C, de préférence comprise entre 250°C et 350°C, et une pression comprise entre 1 et 5 bars.

L'étape de déshydratation de glycérol peut s'effectuer en amont de la réaction d'oxydation catalytique du propylène en présence du gaz d'alimentation comprenant le propylène, ou en aval de la réaction d'oxydation catalytique du propylène en présence du mélange gazeux issu de cette réaction. Elle peut être intégrée directement dans le réacteur d'oxydation ou être réalisée dans un réacteur placé immédiatement en amont ou en aval du réacteur d'oxydation du propylène. La réaction de déshydratation étant faiblement endothermique, il n'est pas nécessaire d'avoir un lit multitubulaire pour cette réaction. Le lit fixe conventionnel peut convenir ainsi qu'une configuration en modules (plaques ou paniers). Les modules présentent l'avantage de pouvoir être chargés et déchargés facilement lorsque le catalyseur est désactivé.

Les catalyseurs qui conviennent sont des matériaux homogènes ou multiphasiques, insolubles dans le milieu réactionnel qui ont me acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse. Les catalyseurs répondant au critère d'acidité H₀ inférieur à +2, peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides.

Avantageusement, les catalyseurs sont choisis parmi les zéolithes, les composites Nafion^{®} (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃. Selon les données de la littérature, ces catalyseurs ont tous une acidité de Hammett H₀ inférieure à +2.

Les catalyseurs préférés sont les zircones sulfatées, les zircones phosphatées, les zircones tungstées, les zircones silicées, les oxydes de titane ou d'étain sulfatées, les alumines ou silices phosphatées.

Ces catalyseurs ont tous une acidité de Hammett H₀ inférieure à +2, l'acidité H₀ peut alors varier dans une large mesure, jusqu'à des valeurs pouvant atteindre -20 dans l'échelle de référence avec les indicateurs de Hammett. Le tableau donné à la page 71 de la publication sur la catalyse acido-basique (C. Marcilly) Vol 1 aux Editions Technip (n°ISBN 2-7108-0841-2) illustre des exemples de catalyseurs solides dans cette gamme d'acidité.

Le glycérol est utilisé pur, ou sous forme de solution aqueuse concentrée ou diluée. Avantageusement, on peut utiliser une solution aqueuse de glycérol de concentration allant de 10% à 100% en poids. Dans le mode de réalisation de l'invention où l'étape de déshydratation de glycérol s'effectue en amont de la réaction d'oxydation catalytique du propylène, on peut utiliser une solution aqueuse de glycérol, de préférence de concentration allant de 10% à 50% en poids, plus particulièrement de 15% à 30% en poids. La concentration ne doit pas être trop élevée afin d'éviter des réactions parasites comme la formation d'éthers de glycérol ou des réactions entre l'acroléine produite et le glycérol. Par ailleurs, la solution de glycérol ne doit pas être trop diluée en raison du coût énergétique induit par l'évaporation de la solution aqueuse de glycérol. Dans le mode de réalisation de l'invention où l'étape de déshydratation de glycérol s'effectue en présence du gaz de réaction issu de l'étape d'oxydation du propylène en acroléine, on peut utiliser du glycérol pur ou une solution aqueuse de glycérol concentrée, ledit gaz de réaction contenant de la vapeur d'eau. De préférence, la concentration de la solution aqueuse de glycérol va de 50% à 100%.

Le glycérol peut être injecté sous forme liquide ou sous forme gazeuse. L'injection sous forme liquide permet de bénéficier de la chaleur latente de vaporisation du glycérol, permettant ainsi de refroidir les gaz issus de l'étape en amont d'oxydation du propylène. Dans ce cas, le catalyseur de déshydratation peut être précédé d'un lit d'inertes sur lequel s'effectue la vaporisation du glycérol. Il peut être injecté sous forme gazeuse à une température inférieure à celle des gaz sortant de la zone d'oxydation, ce qui permet d'accentuer encore l'effet de refroidissement de ces gaz. Par ailleurs, le glycérol peut être injecté sous pression de sorte que la détente du gaz permet une consommation supplémentaire de chaleur.

La réaction de déshydratation du glycérol s'effectue en présence d'oxygène moléculaire qui se trouve dans le mélange gazeux alimentant le réacteur d'oxydation du propylène ou dans le mélange gazeux issu de l'étape d'oxydation du propylène L'oxygène moléculaire peut être présent sous forme d'air ou sous forme d'un mélange de gaz contenant de l'oxygène moléculaire. Selon un mode de réalisation de l'invention, il est possible d'ajouter une quantité supplémentaire d'oxygène moléculaire ou d'un gaz contenant de l'oxygène moléculaire pour l'étape de déshydratation du glycérol. La quantité d'oxygène est choisie de façon à être en dehors du domaine d'inflammabilité en tout point de l'installation. La présence d'oxygène permet de limiter la désactivation du catalyseur de déshydratation par cokage. De plus, l'ajout d'oxygène améliore le rendement de la réaction pour de nombreux systèmes catalytiques.

La réaction d'oxydation catalytique du propylène en acroléine s'effectue selon des conditions connues de l'homme du métier, en faisant passer un mélange gazeux pouvant comporter du propylène, de la vapeur d'eau, un gaz inerte pouvant être de l'azote ou de l'argon et de l'oxygène moléculaire ou un gaz contenant de l'oxygène moléculaire, sur un catalyseur d'oxydation du propylène. Le réacteur d'oxydation est généralement un réacteur multitubulaire en lit fixe. Le réacteur d'oxydation peut être également un échangeur à plaques avec un agencement modulaire du catalyseur tel que décrit dans les documents EP 995491, EP 1147807 ou US 2005/0020851.

Dans le cas où l'oxydation catalytique du propylène s'effectue en présence d'un ballast thermique, telle que décrite par exemple dans le document EP 293 224 A1, permettant la mise en oeuvre d'un débit de propylène plus élevé, le mélange gazeux issu de la réaction possède une chaleur spécifique Cp plus élevée. Le procédé selon l'invention est particulièrement avantageux dans ce cas pour évacuer l'excès de chaleur transporté par les gaz de réaction.

Un mode de réalisation préféré de l'invention consiste à utiliser du propane comme gaz inerte en substitution de tout ou partie de l'azote de l'air. Le propane, grâce à sa chaleur spécifique plus élevée amène plus de calories vers le réacteur, ce qui permet d'effectuer la réaction de déshydratation du glycérol plus facilement. Le gaz issu de l'étape de déshydratation contient alors comme constituants principaux de la vapeur d'eau, du propane, de l'acroléine et de l'oxygène résiduel. Après absorption de l'acroléine, les gaz riches en propane peuvent être recyclés. De préférence, le gaz subit des traitements de purification afin d'éliminer des impuretés qui peuvent être gênantes pour les réactions de déshydratation et d'oxydation, telles que le CO et/ou CO₂, et afin de limiter la concentration de ces impuretés dans la boucle de recycle. Dans ce cas, il est particulièrement avantageux de maîtriser la concentration en argon dans la boucle de gaz du fait de sa très faible chaleur spécifique. Comme techniques de séparation utilisables seules ou en combinaison, on peut citer l'oxydation sélective du CO en CO₂, le lavage aux amines, le lavage à la potasse, les techniques d'adsorption, la séparation membranaire ou la séparation cryogénique.

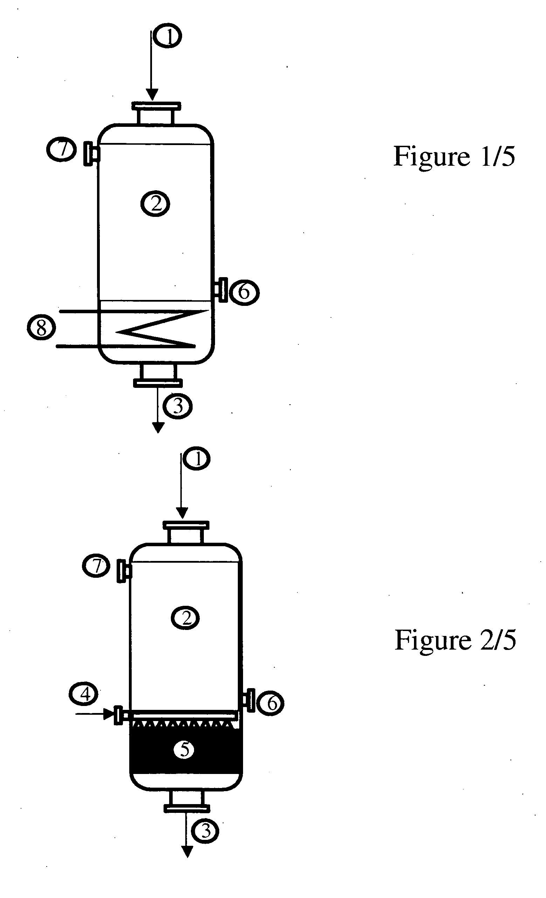

En référence à la Figure 1, dans un procédé conventionnel d'oxydation du propylène en acroléine, on fait passer dans un réacteur multitubulaire du haut vers le bas, un mélange gazeux 1 comportant du propylène, de la vapeur d'eau, de l'azote et de l'oxygène moléculaire, sur un catalyseur 2 d'oxydation du propylène. On obtient, après refroidissement à l'aide d'un échangeur thermique 8, un mélange 3 comprenant les gaz n'ayant pas réagi, l'acroléine produite, et des sous-produits. Des fluides réfrigérants circulent en 6 et 7 de façon à maintenir une température de réaction pouvant être comprise entre 300°C et 320°C. L'échangeur thermique 8 peut être placé directement en aval du lit catalytique, comme sur la Figure 1, ou être installé consécutivement au réacteur d'oxydation.

Conformément à un premier mode de réalisation du procédé selon l'invention, illustré de façon schématique sur la Figure 2, l'échangeur thermique 8 en aval du lit de catalyseur 2 d'oxydation du propylène est remplacé (en tout ou en partie) par une étape de déshydratation de glycérol consistant à faire passer un mélange 4 constitué de glycérol sous forme de solution aqueuse vaporisée et d'oxygène en même temps que le mélange gazeux sortant de la zone d'oxydation, sur un catalyseur 5 de déshydratation du glycérol. On obtient à la sortie un mélange d'acroléine résultant à la fois de la réaction d'oxydation du propylène et de la réaction de déshydratation du glycérol, ainsi que les sous-produits issus de ces deux réactions.

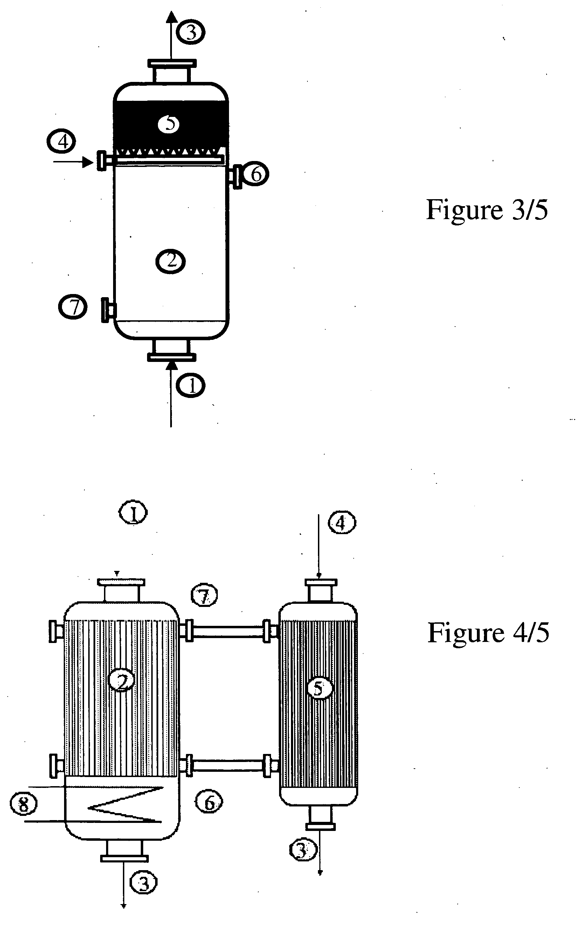

Conformément à un second mode de réalisation du procédé de l'invention, illustré de façon schématique sur la Figure 3, le réacteur d'oxydation est alimenté par le mélange gazeux 1 du bas vers le haut, le catalyseur 5 de déshydratation du glycérol se trouvant dans cette configuration en tête du réacteur. Le changement de catalyseur est ainsi facilité. Le lit de catalyseur de déshydratation pouvant être de type lit fixe conventionnel ou en modules (plaques ou paniers) peut être facilement extrait et remplacé. La régénération du catalyseur peut donc être réalisée facilement en dehors du réacteur.

Conformément à un troisième mode de réalisation du procédé de l'invention, illustré sur la Figure 4, le catalyseur 5 de déshydratation est placé en tout ou en partie dans la chaudière, qui sert à refroidir le fluide thermique circulant en 6 et 7 et qui élimine les calories de la réaction d'oxydation en produisant de la vapeur dans la chaudière.

La réaction endothermique de déshydratation s'effectuant en partie dans la chaudière, il est possible d'éliminer plus de calories, ce qui permet indirectement d'augmenter le débit de propylène dans le réacteur d'oxydation et donc de produire à la fois plus d'acroléine par l'oxydation du propylène et par la déshydratation du glycérol. Dans ce mode de réalisation, il est préférable que les catalyseurs fonctionnent dans des gammes de température voisines.

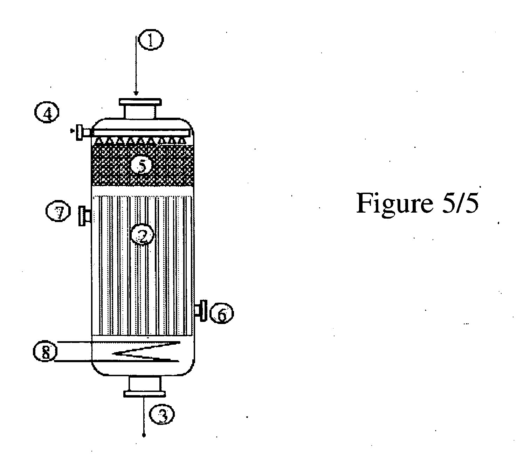

Conformément à un quatrième mode de réalisation du procédé selon l'invention, illustré sur la Figure 5, le catalyseur 5 de déshydratation du glycérol est placé en amont du catalyseur 2 d'oxydation du propylène. Il est nécessaire dans ce cas de chauffer la solution de glycérol à une température élevée pour la vaporiser sur le catalyseur de déshydratation, et pour maintenir les gaz de réaction à une température suffisamment élevée avant d'enter dans la zone catalytique d'oxydation du propylène. Le catalyseur de déshydratation peut être placé dans des modules ou des paniers en tête de réacteur ; il est ainsi facilement changé lorsqu'il est désactivé. Le glycérol 4 peut aussi être co-alimenté avec le mélange gazeux 1 contenant le propylène.. Le catalyseur de déshydratation peut être placé immédiatement au-dessus du lit de catalyseur d'oxydation du propylène.

On peut envisager de mettre en oeuvre une autre réaction endothermique que celle de déshydratation du glycérol pour récupérer de manière efficace la chaleur de réaction d'oxydation. Notamment, h réaction d'oxydéshydratation de propane diol-1,3, ou la déshydratation de propanol-1 ou propanol-2, sont aussi intéressantes sous certains aspects, plus particulièrement si le lit de catalyseur de déshydratation est placé en amont du réacteur d'oxydation du propylène en acroléine. En effet, la déshydratation du propane diol-1,3 peut conduire à de l'alcool allylique qui, à son tour, peut s'oxyder sur le catalyseur d'oxydation du propylène en acroléine. Le propanol-1 ou le propanol-2, peut se déshydrater en propylène et s'oxyder ensuite en acroléine sur le catalyseur d'oxydation.

Les exemples suivants illustrent la présente invention sans toutefois en limiter la portée.

Dans les exemples, les produits formés, acroléine et acide acrylique sont analysés par chromatographie sur colonne capillaire EC-1000 montée sur un chromatographe HP6980 équipé d'un détecteur FID. L'analyse quantitative est effectuée avec un étalon externe.

### Exemple 1 :

On utilise une configuration telle que représentée sur la figure 5, dans laquelle le glycérol est co-alimenté avec le mélange gazeux contenant le propylène, et comportant deux lits de catalyseur.

On utilise un réacteur en pyrex muni d'un fritté pour retenir les catalyseurs. On charge tout d'abord une masse de 6,578 g de catalyseur d'oxydation du propylène en acroléine de référence ACF7 (de Nippon Shokubai), dilué avec 7 ml de carbure de silicium de granulométrie 0,125 mm. Ensuite, on charge plusieurs lits de carbure de silicium (SiC) de manière à séparer les 2 lits de catalyseur et contrôler indépendamment leur température : 2 ml de granulométrie 0,125 mm, puis 7 ml de granulométrie 0,5 mm, puis à nouveau 2 ml de granulométrie 0,125 mm, et finalement 1 ml de granulométrie 0,062 mm.. Ensuite on charge une masse de 1,522 g de catalyseur de déshydratation du glycérol de référence Z1044 (zircone tungstée de Dailchi Kigenso KK), dilué avec 4 ml de carbure de silicium de granulométrie 0,062 mm. Puis, le réacteur est complété jusqu'en haut avec du carbure de silicium de granulométrie 0,125 mm (2ml), 0,5 mm puis 1,19 mm.

Le réacteur est ensuite connecté à l'installation de test. Les températures des deux couches de catalyseur sont régulées de manière indépendante à 260 °C pour la couche supérieure de déshydratation, et à 305 °C pour la couche inférieure d'oxydation.

Le réacteur est alimenté par le haut par un mélange gazeux de propylène / oxygène / hélium-krypton / eau-glycérol. Le mélange gazeux hélium-krypton contient 4,92 % de krypton qui sert d'étalon interne. Le mélange eau-glycérol contient 30 % en poids de glycérol.

Les débits molaires horaires (exprimés en micromoles par heure) des constituants du mélange sont les suivants : 30089 / 55584 / 288393 / eau : 53489 - Glycérol : 4509. Ces conditions représentent un débit molaire total de composés en C₃ (propylène + glycérol) de 34598 micromoles/h.

Les effluents sont collectés en sortie du réacteur par un piège froid à glace et l'acroléine et l'acide acrylique produits sont dosés par analyse chromatographique.

Les effluents sont cumulés dans le piège pendant une durée de 82 minutes. Les gaz non condensables sont analysés pendant la durée du bilan. La quantité d'acroléine produite est de 25302 micromoles/h, et la quantité d'acide acrylique est de 2103 micromoles/h

### Exemple 2 (Comparatif) :

On reproduit l'exemple 1, mais la solution aqueuse de glycérol est remplacée par de l'eau pure. Les débits molaires en micromoles/h des réactifs sont alors : propylène / oxygène / hélium-krypton / eau : 30089 / 55584 / 288393 / 76666.

Les effluents sont cumulés dans le piège pendant une durée de 88 minutes. Les gaz non condensables sont analysés pendant la durée du bilan. La quantité d'acroléine produite est de 20391 micromoles/h, et la quantité d'acide acrylique est de 1157 micromoles/h.

### Exemple 3 (Comparatif) :

On reproduit l'exemple 2, mais en remplaçant le catalyseur de déshydratation par du carbure de silicium. On utilise les mêmes conditions d'alimentation.

Les effluents sont cumulés dans le piège pendant une durée de 75 minutes. Les gaz non condensables sont analysés pendant la durée du bilan. La quantité d'acroléine produite est de 20821 micromoles/h, et la quantité d'acide acrylique est de 1223 micromoles/h.

### Exemple 4 :

On utilise une configuration telle que représentée sur la figure 2, dans laquelle le glycérol est introduit sur un catalyseur de déshydratation en même temps que le mélange gazeux issu de la zone d'oxydation du propylène en acroléine.

On utilise un réacteur en pyrex muni d'un fritté pour retenir les catalyseurs. On charge tout d'abord une masse de 1,538 g de catalyseur de déshydratation de référence Z1044 (zircone tungstée de DaiIchi Kigenso KK), dilué avec 4 ml de carbure de silicium de granulométrie 0,062 mm. Ensuite on charge plusieurs lits de carbure de silicium de manière à séparer les 2 lits de catalyseur et contrôler indépendamment leur température, et permettre l'injection d'une solution aqueuse de glycérol ou du glycérol hydraté entre les deux lits de catalyseur ; on charge 4ml de granulométrie 0,125 mm, puis 7 ml de 0,5 mm et à nouveau 2 ml de 0,125 mm. Ensuite on charge une masse de 6,522 g de catalyseur d'oxydation du propylène en acroléine de référence ACF4 (de Nippon Shokubai), dilué avec 7 ml de carbure de silicium de granulométrie 0,125 mm.

Et finalement, le réacteur est complété jusqu'en haut avec du carbure de silicium de granulométrie 0,125 mm (2ml), 0,5 mm puis 1,19 mm.

Le réacteur est ensuite connecté à l'installation de test. Les températures des deux couches de catalyseur sont régulées de manière indépendante à 260 °C pour la couche inférieure de déshydratation, et à 305 °C pour la couche supérieure d'oxydation.

Le réacteur est alimenté par le haut par un mélange gazeux de propylène / oxygène / hélium-krypton / eau avec les débits molaires horaires suivants (exprimés en micromoles par heure) : 30089 / 55584 / 288393 / 76666. Le mélange gazeux hélium-krypton contient 4,92 % de krypton qui sert d'étalon interne. Un mélange glycérol / eau contenant 80 % en poids de glycérol est alimenté entre les deux lits de catalyseur avec un débit de 4530 / 5794 (micromoles/h). Ces conditions représentent un débit molaire total de composés en C₃ (propylène + glycérol) de 34619 micromoles/h.

Les effluents sont collectés en sortie du réacteur par un piège froid à glace et l'acroléine et l'acide acrylique produits sont dosés par analyse chromatographique.

Les effluents sont cumulés dans le piège pendant une durée de 84 minutes. Les gaz non condensables sont analysés pendant la durée du bilan. La quantité d'acroléine produite est de 25852 micromoles/h, et la quantité d'acide acrylique est de 1170 micromoles/h. Le propylène résiduel est de 2895 micromoles/h.

### Exemple 5 :

On reproduit l'exemple 4, mais en utilisant une solution de glycérol à 95 % en poids (glycérol hydraté).

Les débits molaires horaires (en nicromoles par heure) des constituants du mélange sont les suivants :
propylène / oxygène / hélium-krypton / eau : 30089 / 55584 / 288393 / 76666 pour l'alimentation supérieure et glycérol / eau : 8220 / 2205 (micromoles/h) pour l'alimentation intermédiaire. Ces conditions représentent un débit molaire total de composés en C₃ (propylène + glycérol) de 38309 micromoles/h.

Les effluents sont cumulés dans le piège pendant une durée de 84 minutes. Les gaz non condensables sont analysés pendant la durée du bilan. La quantité d'acroléine produite est de 28099 micromoles/h, et la quantité d'acide acrylique est de 1237 micromoles/h. Le propylène résiduel est de 2856 micromoles/h.

### Exemple 6 (Comparatif) :

On reproduit l'exemple 4 mais en remplaçant le catalyseur de déshydratation par du carbure de silicium et en n'introduisant pas de solution de glycérol.

Les effluents sont cumulés dans le piège pendant une durée de 73 minutes. Les gaz non condensables sont analysés pendant la durée du bilan. La quantité d'acroléine produite est de 22373 micromoles/h, et la quantité d'acide acrylique est de 1150 micromoles/h. Le propylène résiduel est de 2933 micromoles/h.

## Revendications

1. Procédé de préparation d'acroléine par oxydation du propylène, **caractérisé en ce qu'**il comprend une étape de déshydratation de glycérol en présence d'un gaz contenant du propylène.

2. Procédé selon la revendication 1, **caractérisé en ce que** le gaz contenant du propylène est le gaz de réaction issu de l'étape d'oxydation du propylène en acroléine.

3. Procédé selon la revendication 1, **caractérisé en ce que** le gaz contenant du propylène est le mélange gazeux qui alimente le réacteur d'oxydation du propylène.

4. Procédé selon l'une des revendications 1 à 3, **caractérisé en ce que** la réaction de déshydratation est effectuée en phase gaz en présence d'un catalyseur.

5. Procédé selon l'une des revendications 1 à 4, **caractérisé en ce que** l'on ajoute de l'oxygène moléculaire pour l'étape de déshydratation du glycérol.

6. Procédé selon l'une des revendications 1 à 5, **caractérisé en ce que** le glycérol est injecté sous forme liquide ou sous forme gazeuse.

7. Procédé selon l'une des revendications 1 à 6, **caractérisé en ce que** l'on utilise du glycérol pur, ou sous forme de solution aqueuse concentrée ou diluée.

8. Procédé selon l'une des revendications 1 à 7, **caractérisé en ce que** l'oxydation du propylène est réalisée en présence d'un ballast thermique.

9. Procédé selon l'une des revendications 1 à 8, **caractérisé en ce que** l'étape de déshydratation du glycérol est réalisée en partie dans la chaudière qui sert à refroidir le fluide thermique circulant en 6 et 7.

## Claims

1. Process for the preparation of acrolein by oxidation of propylene, **characterized in that** it comprises a stage of dehydration of glycerol in the presence of a propylene-comprising gas.

2. Process according to Claim 1, **characterized in that** the propylene-comprising gas is the reaction gas resulting from the stage of oxidation of the propylene to give acrolein.

3. Process according to Claim 1, **characterized in that** the propylene-comprising gas is a gas mixture which feeds the reactor for the oxidation of the propylene.

4. Process according to one of Claims 1 to 3, **characterized in that** the dehydration reaction is carried out in the gas phase in the presence of a catalyst.

5. Process according to one of Claims 1 to 4, **characterized in that** molecular oxygen is added for the stage of dehydration of the glycerol.

6. Process according to one of Claims 1 to 5, **characterized in that** the glycerol is injected in the liquid form or in the gas form.

7. Process according to one of Claims 1 to 6, **characterized in that** use is made of pure glycerol or glycerol in the form of a concentrated or dilute aqueous solution.

8. Process according to one of Claims 1 to 7, **characterized in that** the oxidation of the propylene is carried out in the presence of a thermal ballast.

9. Process according to one of Claims 1 to 8, **characterized in that** the stage of dehydration of the glycerol is carried out in part in the boiler which is used to cool the heat-exchange fluid circulating in 6 and 7.

## Patentansprüche

1. Verfahren zur Herstellung von Acrolein mittels Oxidation von Propylen, **dadurch gekennzeichnet, dass** es einen Schritt der Absolutierung von Glycerin in Gegenwart eines Gases, das Propylen enthält, umfasst.

2. Verfahren nach Anspruch 1, **dadurch gekennzeichnet, dass** das Gas, das Propylen enthält, das Reaktionsgas ist, das aus dem Schritt der Oxidation des Propylens zu Acrolein hervorgeht.

3. Verfahren nach Anspruch 1, **dadurch gekennzeichnet, dass** das Gas, das Propylen enthält, das Gasgemisch ist, das den Reaktor zur Oxidation des Propylens speist.

4. Verfahren nach einem der Ansprüche 1 bis 3, **dadurch gekennzeichnet, dass** die Absolutierungsreaktion in der Gasphase in Gegenwart eines Katalysators bewirkt wird.

5. Verfahren nach einem der Ansprüche 1 bis 4, **dadurch gekennzeichnet, dass** molekularer Sauerstoff für den Schritt der Absolutierung des Glycerins zugesetzt wird.

6. Verfahren nach einem der Ansprüche 1 bis 5, **dadurch gekennzeichnet, dass** das Glycerin in flüssiger Form oder gasförmig eingespritzt wird.

7. Verfahren nach einem der Ansprüche 1 bis 6, **dadurch gekennzeichnet, dass** reines Glycerin oder Glycerin in der Form einer verdünnten oder konzentrierten wässrigen Lösung verwendet wird.

8. Verfahren nach einem der Ansprüche 1 bis 7, **dadurch gekennzeichnet, dass** die Oxidation des Propylens in Gegenwart eines thermischen Ballaststoffs durchgeführt wird.

9. Verfahren nach einem der Ansprüche 1 bis 8, **dadurch gekennzeichnet, dass** der Schritt der Absolutierung des Glycerins zum Teil in dem Kessel durchgeführt wird, der zum Abkühlen der Heizflüssigkeit dient, die in 6 und 7 zirkuliert.
